# EUROPEAN PATENT SPECIFICATION

(11) **EP 3 727 023 B1**
(45) Date of publication and mention of the grant of the patent: **18.11.2026**
(21) Application number: 18822358.0
(22) Date of filing: 20.12.2018
(51) Int. Cl.: A23K 20/158, A23K 20/163, A23K 50/75

(54) **ANIMAL FEED MATERIAL**
TIERFUTTERMATERIAL
MATIÈRE PREMIÈRE POUR ALIMENTS POUR ANIMAUX

(30) Priority: 22.12.2017 BE 201705984
(43) Date of publication of application: 28.10.2020
(73) Proprietor: Nutriad International, 2300 Turnhout (BE)
(72) Inventor: GOOSSENS, Tim, 9200 Dendermonde (BE); VAN IMMERSEEL, Filip Florimond Magdalena, 9200 Dendermonde (BE); KWAKKEL, René, 9200 Dendermonde (BE); MOQUET, Pierre Clément Antoine, 9200 Dendermonde (BE); ONRUST, Lonneke, 9200 Dendermonde (BE)
(74) Representative: Germain Maureau
(86) International application number: PCT/EP2018/086173
(87) International publication number: WO 2019/122112

(56) References cited:
- WO-A1-2007/124949
- WO-A1-2016/055651
- WO-A1-2018/174141
- GB-A- 2 466 041
- US-A- 5 089 271
- US-A1- 2003 175 326
- LI MUFANG ET AL: "Highly hydrophilic and anti-fouling cellulose thin film composite membrane based on the hierarchical poly(vinyl alcohol-co-ethylene) nanofiber substrate", CELLULOSE, SPRINGER NETHERLANDS, NETHERLANDS, vol. 22, no. 4, 16 June 2015 (2015-06-16), pages 2717 - 2727, XP035515892, ISSN: 0969-0239, [retrieved on 20150616], DOI: 10.1007/S10570-015-0682-3

## Description

### Field of the invention

The present invention relates to the field of livestock animal feed, in particular to a feed material that is effective in prevention and/or treatment of pathogenic infections in livestock and/or in increasing efficiency in livestock production.

### Background of the invention

The growing demand for livestock products worldwide requires novel strategies that increase the efficiency of livestock production. Optimization of nutrient utilization is an essential element in such strategies. Consumer and governmental focus on food safety, lowering environmental impact, better animal welfare and prudent antimicrobial use generate additional demands on livestock production.

One particular area of attention in livestock production is the eradication of pathogens, such as *Salmonella* and other pathogenic bacteria. Salmonella is one of the most important causes of foodborne infections in humans, mainly due to the consumption of poultry meat or contaminated eggs. The agricultural sector attempts to reduce the number of *Salmonella* infections by different measures such as vaccination, intensive hygienic measures and/or administration of antibiotics, probiotics, acidifiers, or short- and medium-chain fatty acids and salts thereof.

Recently, the bacteriostatic effects of volatile short-chain fatty acids on gram-negative bacteria have attracted attention. The group of volatile short-chain fatty acids consists of biodegradable weak organic acids that are able to eliminate pathogenic microorganisms without significantly affecting the intestinal microflora. It has been shown that volatile short-chain fatty acids may inhibit the growth of *Escherichia coli* hemolytic strains by 50 percent. A number of short chain fatty acid compositions have been developed for use as a feed additive in order to achieve reductions of pathogens in animals.

EP1354520 describes a feed additive which is a microcapsule comprising n-butyric acid in a matrix comprising a lipidic structure (wax) and its preparation by spray-cooling. According to EP1354520 micro-encapsulation of butyric acid is useful in particular to counteract the difficulties associated with the volatility and rancid smell of butyric acid, which create difficulties in the handling as a feed additive. It is also suggested in EP1354520 that the formulation is stable against gastric degradation.

F. Van Immerseel et al. (2005 Poultry Science 84: 1851-1856) compared the effect of butyric acid with butyric acid embedded in a fat matrix when used as a feed additive for poultry. They reported that colonization of caeca by *Salmonella* and fecal shedding of *Salmonella* was significantly lower in the group which was administered butyric acid embedded in a fat matrix.

WO2007/124949 describes the use of 3-hydroxybutyric acid and poly-3-hydroxybutyric acid compounds as feed additives.

BE1023491 describes a feed additive comprising butyric acid in a wax matrix comprising microcrystalline wax, and its production by melt extrusion.

GB2466041A discloses coated granules comprising butyrate salt for use in animal feed for improving health and resistance against bacterial intrusion in the gastrointestinal tract.

It is an object of the present invention to provide compounds possessing improved effectiveness in preventing or treating pathogenic infections in livestock and/or in increasing efficiency in livestock production.

### Summary of the invention

The present inventors have surprisingly found that these objectives can be met with certain esters of polysaccharide and butyric acid.

It has been found that enteral administration of polysaccharide butyryl esters of the present invention to livestock animals results in increased butyrate concentrations in the lower intestinal tract. The present inventors assume this correlates to the effectiveness of the polysaccharide butyryl esters on pathogenic presence in the lower intestinal tract and feces that has been observed during in vivo trials. It was also surprisingly found that administration of the polysaccharide butyryl esters resulted in superior growth performance results compared to other butyrate formulations or products. The polysaccharide butyryl esters in accordance with the present invention provide the highly desirable combination of improved average daily weight gain (ADG) and average daily food intake (ADFI) combined with decreased feed conversion ratio (FCR) and mortality during the supplementation period without showing adverse effects during the finisher period.

Butyric acid is presumed to stimulate the growth of the intestinal villi and/or modifies the development of gastro-enteric micro-organisms. It is also believed that butyric acid can downregulate expression of genes involved in Salmonella invasion at low doses. Other beneficial effects which the inventors envisage upon use of the polysaccharide butyryl esters of the present invention may comprise an improved retention of intestinal content in the small intestine, an improved digestion/absorption of methionine and/or a more diverse microbial population in the lower gastro-intestinal tract.

The origin of the butyric acid concentration increase may be a prebiotic effect (for example, but not limited to a prebiotic effect on butyric acid producing bacteria), direct release of butyric acid resulting from degradation of the polysaccharide butyryl ester, any other mechanism resulting in a butyric acid concentration increase or a combination thereof. Without wishing to be bound by any theory, the present inventors believe that the effects observed for the polysaccharide butyryl esters of the present invention may also correlate with an increased presence of microorganisms capable of polysaccharide fermentation in the lower gastro-intestinal tract compared to the upper gastro-intestinal tract.

Hence the present invention provides the uses of polysaccharide butyryl esters. for preventing or treating pathogenic infections in livestock and/or in increasing efficiency in livestock production.

These and other aspects of the invention will become apparent on the basis of the following detailed description and the appended examples.

### Detailed description of the invention

A first aspect of the invention concerns a polysaccharide butyryl ester for use in a prophylactic or curative method of treatment.

Butyric acid is a volatile short-chain fatty monocarboxylic acid having the molecular formula CH₃-CH₂-CH₂-COOH. The terms "butyric acid" and "butyrate" as used herein are used interchangeably and should be construed to denote the protonated (acid, butyric acid) and deprotonated (conjugate base, butyrate) forms respectively. The skilled person will understand that butyric acid, as it is a weak acid, is normally present in both protonated and deprotonated form when dissolved in an aqueous medium, the concentration of each form depending on the pH of the medium. The acid form can be absorbed by the intestinal walls and by the cellular membranes of micro-organisms.

As used herein, the term "polysaccharide butyryl ester" refers to compounds that, generally stated, comprise a polysaccharide molecule as the central portion, which polysaccharide molecule is derivatized/substituted with a plurality of butyric acid molecules through the formation of ester linkages between the carboxylic acid moiety of the butyric acid molecule and a hydroxyl group of the polysaccharide.

As used herein, the term "polysaccharide" refers to polymers comprising a backbone comprising monosaccharide repeating units and/or derivatized monosaccharide repeating units, typically cyclic pentoses, in particular C₅ aldoses or ketoses, or cyclic hexoses, in particular C₆ aldoses or ketoses. Non limiting examples of C₅-C₆ aldoses include allose, altrose, glucose, mannose, gulose, idose, galactose, talose, ribose, arabinose, xylose, lyxose. Non limiting examples of C₅-C₆ ketoses include ribulose, xylulose, fructose, sorbose and tagatose. As used herein, the term "monosaccharide derivatives" refers to any chemically or enzymatically modified monosaccharide unit.

The polysaccharide may be a homopolysaccharide or heteropolysaccharide.

The polysaccharide may be a modified or unmodified. The polysaccharide may be selected from the group consisting of starches, modified starches, amylopectin, modified amylopectin, amylose, modified amylose, chitosan, chitin, guar gum, modified guar gum, locust bean gum, tara gum, konjac gum, konjac flour, fenugreek gum, mesquite gum, aloe mannans, modified cellulose, oxidized polysaccharides, sulfated polysaccharides, cationic polysaccharides, arabic gum, karaya gum, xanthan, kappa, iota or lambda carrageenans, agar-agar, alginates, callose, laminarin, chrysolaminarin, xylan, mannan, galactomannan, hemicellulose, pectin, arabinoxylan, xanthan gum, nigeran, isolichenan, laminaran, lichenan, glycogen, pullulan, dextran, pustulan, inulin, grass levans, carrageenan, galactocarolose, rhodymenan, fucoidan, agarose, porphyran, alginic acid, keratosulphate, chondroitin, chrondroitin sulphates, heparin and cellulose. In accordance with the invention, the polysaccharide is cellulose.

For the purposes of the present invention, the polysaccharide butyryl ester may be formed chemically, enzymatically, fermentatively, through biosynthesis by a natural or genetically modified organism, etc. Typically, in accordance with the invention, the polysaccharide butyryl ester is produced by esterification of a polysaccharide such as acid-catalyzed esterification.

The amount of substituent groups on the (anhydro) monosaccharide units of polysaccharides can be designated by weight percent or by the average number of substituent groups attached to the ring, a concept known to polysaccharide chemists as "degree of substitution" (D.S.). In case of a C₆ monosaccharide, if all three available positions on each unit are substituted, the D.S. is designated as 3; if an average of two on each ring are substituted, the D.S. is designated as 2, etc. In analogy the amount of substituent groups on the polysaccharides can be can be expressed as a percentage of the available positions that are substituted.

In one embodiment of the invention, the polysaccharide butyryl ester has an average number of butyryl groups per monosaccharide unit (D.S.) within the range of 0.1-4.

In a particularly preferred embodiment of the invention, the polysaccharide butyryl ester is a cellulose butyryl ester having an average number of butyryl groups per monosaccharide unit (D.S.) within the range of 0.1-4, preferably 1-3.5, preferably 1.75-3.25, preferably 2.25-3, preferably 2.5-2.95.

In embodiments of the invention, the polysaccharide butyryl ester is a cellulose butyryl ester characterized by a butyryl content, expressed as the percentage of the available polysaccharide hydroxyl groups that is substituted, within the range of 5-80 %, preferably 25-70 %, 40-60 %, or 50-56 %.

In accordance with the invention, the polysaccharide butyryl ester is a cellulose butyryl ester comprising acetyl ester groups.

In accordance with the invention, the polysaccharide butyryl ester a number average molar mass (Mₙ) in the range of 2,000-1,000,000 g/mol, preferably withing the range of 5,000-500,000 g/mol, 7,000-250,000 g/mol, 10,000-100,000 g/mol, 12,000-50,000 g/mol, 13,000-25,000 g/mol, or 15,000-17,000 g/mol. In embodiments of the invention, the polysaccharide butyryl ester may be characterized by a number average molar mass (Mₙ) of at least 2,000 g/mol, preferably at least 4,000 g/mol, preferably at least 8,000 g/mol, preferably at least 12,000 g/mol. Furthermore, in embodiments of the invention, the polysaccharide butyryl ester may be characterized by a number average molar mass mass (Mₙ) of less than 1,000,000 g/mol, preferably less than 500,000 g/mol, preferably less than 250,000 g/mol, preferably less than 100,000 g/mol, preferably less than 70,000 g/mol, preferably less than 65,000 g/mol, preferably less than 30,000 g/mol, preferably less than 25,000 g/mol, preferably less than 17,000 g/mol. The number average molar mass (Mₙ) may be determined by suitable methods known to the person skilled in the art such as osmosis, static light scattering, sedimentation equilibrium, gel permeation chromatography, viscosimetry, sedimentation velocity, dynamic light scattering, end group analysis etc. A preferred method to determine the number average molar mass (Mₙ) is gel permeation chromatography.

It is described compositions comprising the polysaccharide butyryl ester of the invention as a feed grade composition. As used herein, the term "feed grade" means suitable for consumption by an animal, in particular livestock. In an embodiment it means that the composition has been determined to be safe, functional and suitable for its intended use in animal food. For example, it is handled and labeled appropriately, and/or conforms to the appropriate regulations governing the use of the composition in animal food in the relevant jurisdiction.

The feed grade composition may comprise at least one further ingredient selected from the groups consisting of feed additives, feed grade formulating aids or excipients, and nutritional components.

The choice and the (relative) amounts of such additional ingredients will depend on the precise form and purpose of the feed grade composition. The feed grade composition may be a feed additive or feed material. The feed grade composition may also be a feed pre-mix or a feed or fodder that is ready to use. Unless indicated otherwise, the terms "feed additive" and "feed material" are used herein interchangeably, as generally referring to compositions that contain the polysaccharide butyryl ester in high concentrations, which compositions are designed and intended to be mixed with feed or fodder to provide the polysaccharide butyryl ester in adequate quantities/dosages. It is to be understood that these terms herein do not refer to legal definitions used in the context of animal feed regulations, which legal definitions may diverge among jurisdictions and/or change over time.

The feed grade composition is a feed material comprising the polysaccharide butyryl ester in association with at least one other feed grade material. The feed material may be provided comprising 0.1-80 wt.%, preferably 0.5-60 wt.%, 1-50 wt.%, 2-40 wt.%, 3-30 wt.%, 4-20 wt.%, 5-15 wt.% of the polysaccharide butyryl ester and one or more other feed grade materials.

A feed material as defined herein is provided, wherein, said at least one other feed grade material is selected from the group of technological additives, sensory additives, nutritional additives, zootechnical additives, coccidiostats and histomonostats.

Examples of technological additives that can suitably be combined with the polysaccharide butyryl ester of the present invention include preservatives, antioxidants, emulsifiers, stabilisers, thickeners, gelling agents, binders, substances for control of radionucleide contamination, anticaking agents, acidity regulators, silage additives and denaturants.

Examples of sensory additives that can suitably be combined with the polysaccharide butyryl ester of the present invention include colourants and flavoring compounds. Colourants should be construed broadly and may indicate substances that add or restore colour in feed, substances which when fed to animals add colours to food of animal origin and/or substances which favourably affect the colour of ornamental fish or birds.

Examples of nutritional additives that can suitably be combined with the polysaccharide butyryl ester of the present invention include vitamins, pro-vitamins and chemically well-defined substances having similar effect; compounds of trace elements; amino acids, their salts and analogues; urea and its derivatives.

Examples of zootechnical additives that can suitably be combined with the polysaccharide butyryl ester of the present invention include digestibility enhancers, gut flora stabilisers and substances which favourably affect the environment. The at least one other feed grade material comprised in the feed material may be a binder, an anticaking agent, a stabilizing agent, a carrier and/or a preservative. The at least one other feed grade material may be selected from the group of a yeast product, a clay, a salt of a fatty acid, ilica, sepiolite, bentonite, clinoptilolite, guar gum, xantham gum, formic acid, sodium formate, calcium formate, acetic acid, calcium acetate, sodium propionate, calcium propionate, lactic acid, calcium lactate, Tocopherol- rich extracts from vegetableoils, and wheat bran. A yeast product as used herein should be construed broadly and may indicate yeast and its derivative products, such as inactivated dry yeast, yeast cell walls, autolysates, or nucleotides, obtained from e.g. *Saccharomyces cerevisiae, Saccharomyces carlsbergensis, Kluyveromyces lactis, Kluyveromyces fragilis, Torulaspora delbrueckii,* etc. The polysaccharide butyryl ester may be present in a feed material in an amount of up to 50 wt.%, based on the total weight of the composition, preferably up to 40 wt.%, up to 30 wt.%, up to 25 wt.%, up to 20 wt.%, up to 18 wt.%, up to 15 wt.%, up to 10 wt.%, or up to 5 wt.% .

The feed grade composition may be a livestock feed, also referred to as fodder, comprising the polysaccharide butyryl ester as described herein and one or more animal feed ingredients. As is understood by those skilled in the art, the term "feed ingredient" as used herein refers to the feed components that provide the common nutrients needed for normal growth and development of the animal, the key nutrients being amino acids, carbohydrates, lipids, vitamins and minerals. Typically, feed ingredients are broadly classified into protein sources, energy sources, fat sources and mineral sources.

Thus, a livestock feed as defined herein is described, wherein the one or more feed ingredients are selected from the group of protein sources, energy sources, fat sources and mineral sources. Suitable examples of protein sources include soybean meal, rapeseed meal, palm kernel meal, sunflower meal, peas, beans, lupins, fish meal, poultry meal and blood plasma. Suitable examples of energy sources include corn, wheat, barley and rice. Suitable examples of fat sources include fish oil, tallow, corn oil, soy oil, rice bran oi, palm oil and canola oil. Suitable examples of mineral sources include calcium, magnesium, phosphorus, potassium, sodium, copper, selenium, zinc, iron, manganese, iodine, cobalt.

A livestock feed as defined herein is described, comprising the polysaccharide butyryl ester in an amount of at least 0.0001 wt.%, based on the total weight of the composition, preferably at least 0.001 wt.%, at least 0.005 wt.%, at least 0.01 wt.%, at least 0.025 wt.%, at least 0.05 wt.% or at least 0.1 wt.%. The polysaccharide butyryl ester may be present in a livestock feed in an amount of up to 10 wt.%, based on the total weight of the composition, preferably up to 5 wt.%, up to 2 wt.%, up to 1 wt.%, up to 0.5 wt.% or up to 0.1 wt.%.

A feed grade composition may have a free acid content of less than 5% by weight of the composition, preferably less than 2% by weight, preferably less than 1% by weight, preferably less than 0.5% by weight, preferably less than 0.1% by weight, preferably less than 0.05% by weight, preferably less than 0.01% by weight. The free acid content may be determined by suitable methods known to the person skilled in the art.

A feed grade composition may have a water content of less than 5% by weight of the composition, preferably less than 2% by weight, preferably less than 1% by weight, preferably less than 0.5% by weight, preferably less than 0.1% by weight, preferably less than 0.05% by weight, preferably less than 0.01% by weight. The water content may be determined by suitable methods known to the person skilled in the art such as Karl-Fischer titration.

The feed grade compositions comprising a polysaccharide butyryl ester as described herein above, may be provided in powder, compacted, granulated or pelletized form. In preferred embodiments the polysaccharide butyryl ester is homogenously distributed throughout the feed grade composition.

The feed grade composition may be provided in compacted, granulated or pelletized form and the polysaccharide butyryl ester is distributed throughout the primary particles making up the food grade composition.

The feed grade composition may be provided in compacted, granulated or pelletized form wherein the primary particles making up the food grade composition do not comprise distinguishable layers or phases. The feed grade composition may also be provided in compacted, granulated or pelletized form wherein the primary particles making up the food grade composition which comprises polysaccharide butyryl ester do not comprise a coating consisting of or comprising the butyryl ester in amounts of more than 70 %, more than 50 % or more than 20 wt.% by total weight of the coating.

A second aspect which is not part of the present invention, it is described a method of treating an animal by administering a polysaccharide butyryl ester as defined herein. Typically, as will be understood on the basis of the foregoing, such methods involve enteral, in particular oral, administration of the polysaccharide butyryl ester to an animal, preferably in admixture with the animal's feed, with the purpose of affecting the animal's physiological state, in particular with the purpose of improving the animal's intestinal health, condition and/or performance. These methods may thus have a prophylactic or curative effect.

In accordance with the invention, the animal is preferably a livestock animal, including avian species, aquatic species, and mammalian species. Examples of avian species include poultry species, such as turkey, duck and chicken. Examples of aquatic species include fish species, such salmon, trout, tilapia, catfish and carp, and crustacean species, including shrimp and prawn. Examples of mammalian species include ruminant species, such as sheep, goat, and cattle, and non-ruminant species, such as horses, pigs and swine. In a preferred embodiment of the invention, the animal is selected from the group consisting of monogastric animals with hindgut fermentation. In other preferred embodiments the animal is selected from the group consisting of chicken, pigs, horses, calves, goats, sheep, rabbits, dogs, cats and fish. In more preferred embodiments the animal is selected from the group consisting of chicken, pigs, horses calves, goat sheep and rabbits, more preferably from the group consisting of chicken, pigs, horses and calves, more preferably from the group consisting of chickens and pigs, most preferably chickens.

The animal to be treated can be an animal that is in the weaning period, an animal that is in the starter period, an animal that is in the grower period or an animal that is in the finisher period. In preferred embodiments, the animal is an animal that is in the starter period.

The method of treatment comprises feeding the animal a feed or fodder, typically a feed or fodder as defined herein elsewhere, comprising the polysaccharide butyryl ester. The optimal treatment regimen may depend on the species treated and/or the effect aimed at and, based on the present teachings, it will be within the capabilities of those skilled in the art to determine appropriate treatment regimens. The treatment will comprise the enteral, e.g. oral, administration of the polysaccharide butyryl ester in a dose of at least 0.0001 wt.%, based on the total weight of the animal feed consumed by the animal in 24h, preferably at least 0.001 wt.%, at least 0.005 wt.%, at least 0.01 wt.%, at least 0.025 wt.%, at least 0.05 wt.% or at least 0.1 wt.%. A treatment as defined herein is provided, comprising the enteral, e.g. oral administration of the polysaccharide butyryl ester in a dose of of up to 10 wt.%, based on the total weight of the animal feed consumed by the animal in 24h, preferably up to 5 wt.%, up to 2 wt.%, up to 1 wt.%, up to 0.5 wt.% or up to 0.1 wt.%.

The polysaccharide butyryl ester may be administered in such doses at least once a week, preferably at least once every three days, more preferably at least once every two days, most preferably once a day.

The methods may be carried out for a variety of reasons, as will be evident on the basis of the foregoing, in particular for improving and/or maintaining the health of the animal and/or for improving the animal's performance.

Hence, a method is described as defined herein, wherein said method is non-therapeutic. Hence, a method is provided as defined herein, wherein the animal to be treated is an animal that is in good or normal health. Said method may be aimed at decreasing the feed conversion ratio, increasing the life weight and/or increasing the average daily gain.

The method may be aimed at increasing the life weight at a given time, such as slaughter or day 35, preferably day 35, by more than 1%, preferably more than 2%, preferably more than 4%, preferably more than 6%. In a preferred embodiment the method is aimed at increasing the life weight at a given time by 1-12%, preferably 2-6%.The increase in life weight at a given time can be determined by the skilled person by routine experimentation, e.g. an *in vivo* experiment using a control group.

The method may also be aimed at decreasing the feed conversion ratio, calculated over a given period, such as the supplementation period or lifetime, preferably the supplementation period, by more than 1%, preferably more than 2%, preferably more than 4%, preferably more than 6%. In a preferred embodiment the method is aimed at decreasing the feed conversion ratio calculated over a given period by 1-12%, preferably 2-6%. The decrease in the feed conversion ratio calculated over a given period can be determined by the skilled person by routine experimentation, e.g. an *in vivo* experiment using a control group.

The method may also be aimed at increasing the average daily gain, calculated over a given period, such as the supplementation period or lifetime, preferably the supplementation period, by more than 1%, preferably more than 2%, preferably more than 4%, preferably more than 6%. In a preferred embodiment the method is aimed at increasing the average daily gain calculated over a given period by 1-12%, preferably 2-6%. The increase in the average daily gain calculated over a given period can be determined by the skilled person by routine experimentation, e.g. an *in vivo* experiment using a control group.

Said method may be carried out with the aim of improving or maintaining the animal's health.

Hence, the animal to be treated may be an animal suffering from or at risk of suffering from a condition or pathology. Furthermoremethodsmay be for curative and/or prophylactic treatment of a condition or pathology in an animal.

More in particular, in embodiments of the invention, the cellulose butyryl ester for use in accordance with the inventionis intended for an animal suffering from or at risk of suffering from pathogen infection, preferably intestinal pathogen infection, more preferably caecal and/or colonic pathogen infection. In embodiments of the invention, it is intended for treating and/or preventing pathogen infection, preferably intestinal pathogen infection, more preferably caecal and/or colonic pathogen infection. In accordance with the invention, said pathogen may be selected from bacteria, eimeria, viruses and fungi, more preferably said pathogen is selected from bacteria, most preferably from *Clostridium acetobutylicum, Escherichia coli, Streptococcus cremoris, Lactococcus lactis, Lactococcus cremoris, Clostridium perfringens, Campylobacter jejuni, Campylobacter coli, Lawsonia intracellullaris, Brachyspira hyodysenteriae, Enterococcus caecorum, Streptococcus suis, Salmonella enteritidis* and combinations thereof, preferably *Clostridium perfringens, Campylobacter jejuni, Campylobacter coli, Lawsonia intracellullaris, Brachyspira hyodysenteriae, Enterococcus caecorum, Streptococcus suis, Salmonella enteritidis* and combinations thereof.

The animal to be treated may be an animal suffering from or at risk of suffering from disturbances in the intestinal flora, in particular disturbances in the caecal and/or colonic microflora. In embodiments of the invention a cellulose butyryl ester is provided as defined herein for improving the intestinal microflora of an animal and/or for maintaining a healthy intestinal flora in an animal, in particular for improving the caecal and/or colonic microflora and/or for maintaining a healthy caecal and/or colonic microflora. In embodiments of the invention, cellulose butyryl esters as defined herein are provided that result in and/or are aimed at increasing the microbial count of bacteria from the *Lactobacillus* or *Bifidobacterium* genus in the gastro-intestinal tract, preferably the lower gastro-intestinal tract, preferably the caeca or colon. In embodiments of the invention, cellulose butyryl esters as defined herein are provided that result in and/or are aimed at increasing the ratio of microbial counts of bacteria from the *Lactobacillus* or *Bifidobacterium* genus compared to microbial counts of bacteria from the Enterobacteriaceae family in the gastro-intestinal tract, preferably the lower gastro-intestinal tract, preferably the caeca or colon. In embodiments of the invention, cellulose butyryl esters as defined herein are provided that result in and/or aimed at increasing the ratio of microbial counts of bacteria from the *Lactobacillus* or *Bifidobacterium* genus compared to microbial counts of bacteria from the *Salmonella* genus in the gastro-intestinal tract, preferably the lower gastro-intestinal tract, preferably the caeca or colon. In embodiments of the invention, cellulose butyryl esters as defined herein are provided that result in and/or aimed at improving the intestinal microflora comprising increasing the ratio of microbial counts of bacteria from the *Lactobacillus* or *Bifidobacterium* genus compared to microbial counts of bacteria from the *Salmonella* genus in the gastro-intestinal tract, preferably the lower gastro-intestinal tract, preferably the caeca or colon wherein the ratio is approximated by determining the microbial count of one species of bacteria representative of each genus and calculating the ratio using the microbial count of the representative bacteria. In accordance with such embodiments, reducing or increasing the microbial count or ratio of microbial counts should be construed broadly and could for example be understood to mean one or more of the following:
- A reduction or increase in the number of colony forming units (CFU) determined using a suitable method known to the person skilled in the art, measured 1-10, preferably 1-5, preferably 3 days after treatment is started and compared to a group of animals which were not treated,
- A reduction or increase in the number of colony forming units (CFU) determined using a suitable method known to the person skilled in the art, measured in the same animal(s) before treatment and 1-10, preferably 1-5, preferably 3 days after treatment is started, or
- any other method known to the person skilled in the art to determine the effect of feed supplementation with a polysaccharide butyryl ester in accordance with the invention.

A cellulose butyryl ester of the invention may be used as follows:
- providing a first animal feed component,
- providing a polysaccharide butyrate ester, and
- mixing the first animal feed component with said polysaccharide butyrate ester to a homogeneous blend.

In an embodiment said method comprises:
- providing a first animal feed component,
- providing a polysaccharide butyrate ester,
- mixing the first animal feed component with said polysaccharide butyrate ester to a homogeneous blend, and
- pelletizing the homogeneous blend.

For a proper understanding of this document and in its claims, it is to be understood that the verb "to comprise" and its conjugations is used in its non-limiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one". The following examples are offered for illustrative purposes only, and are not intended to limit the scope of the present invention in any way.

### Brief description of the figures

Figure 1: Butyrate concentrations measured in example 1 - Ileum inoculum.
Figure 2: Butyrate concentrations measured in example 1 - Colon inoculum.
Figure 3: Butyrate concentrations measured in example 1 - Caecum inoculum.
Figure 4: Butyrate concentrations measured in example 2 - Colon.

### Examples

### Example 1: In vitro fermentation assay

In order to assess the capacity of the polysaccharide butyryl esters of the present invention to increase the butyrate concentration in the gastro-intestinal tract (GIT) of animals, an *in vitro* fermentation assay was performed wherein GIT bacteria of broiler chickens were inoculated together with a productas described above, a positive control and a negative control.

### Medium

1 liter medium consists of 0.6 g KCL, 0.6 g NaCl, 0.2 g CaCl2·2H₂O, 0.5 g MgSO₄·7H₂O, 1.5 g Pipes buffer, 0.54 g NH₄Cl, 1.0 g trypticase, 1 ml resazurin solution (0.2 g resazurin per 200 ml distilled water), 10 ml 'Trace Mineral Solution', 12ml vitamin/phosphate solution, 10 ml Haemin Solution (0.1 g Haemin/ 1 L distilled water), 4mg/ml NaHCO₃ and 1mg/ml cysteine HCl.
- Trace Mineral Solution
   0.025 g CuCl·2H₂O, 0.020 g FeSO₄·7H₂O, 0.025 ZnCl₂, 0.025 g CuCl₂·H₂O, 0.050 g CoCl₂·6H₂O, 0.050 g SeO₂, 0.250 g NiCl₂·6H₂O, 0.250 g Na₂MoO₄·2H₂O, 0.0314 g NaVO₃, 0.250 H₃BO₃ / 1 L 0.02 M HCl dissolution
- Vitamin/phosphate solution
   0.0204 g biotin, 0.0205 g folic acid, 0.1640 g calcium D-phantothenate, 0.1640 g nicotinamide, 0.1640 riboflavin, 0.1640 g thiamin HCl, 0.1640 g pyridoxine HCl, 0.0204 g para-amino benzoic acid, 0.0205 cyanocobalamin (vitamin B12) per 1 L distilled water containing 54.7 g KH₂PO₄,

### Inoculum

Intestinal sample of ileum, colon and caecum of the GIT of a 4-week old broiler Ross 408 was collected. Inoculum was prepared the same way for every segment. Immediately after dissection the samples were placed under anaerobic conditions with a temperature of 37°C (anaerobic chamber, Ruskinn Technology, Bridgend, UK). The content was weighed of every segment and diluted in a ratio of 1:9 with pre-warmed (37°C) anaerobic, sterile phosphate buffered saline. After homogenization, test products were added and the diluted material was inoculated in a ratio of 1:10 in the above-described medium.

### Test material

As shown in the below table, three different materials were tested for their capacity to increase the butyrate concentration in the GIT. All tests were performed at 0.5% w/v end concentration.

In accordance with the invention, cellulose acetate butyrate is used. Xylo-oligosaccharides were included as a positive control because of their known prebiotic effects on butyrate-producing bacteria. In order to assess the effect of the cellulose backbone on the butyrate concentration, a negative control consisting of cellulose was also tested.

| **Test** | **Product** | **Supplier** | **Specifications** |
|---|---|---|---|
| 1 | Cellulose acetate butyrate (CAB) | Acros Organics^{®} | 50-54% butyryl content |
| | | | <4% acetyl content |
| 2 | Xylo-oligosaccharides (XOS) | Longlive^{®} | 35% XOS |
| 3 | Cellulose | Sigma-Aldrich ^{®} | |

### Measurement butyrate concentration

Fluid samples were collected at timepoints 0h, 4h 6h and 24h after inoculation for analysis of butyrate concentration with HPLC-UV. Extraction protocol and equipment was used as described by De Baere et al. (Journal of Pharmaceutical and Biomedical Analysis 80 (2013) 107- 115.

### Results

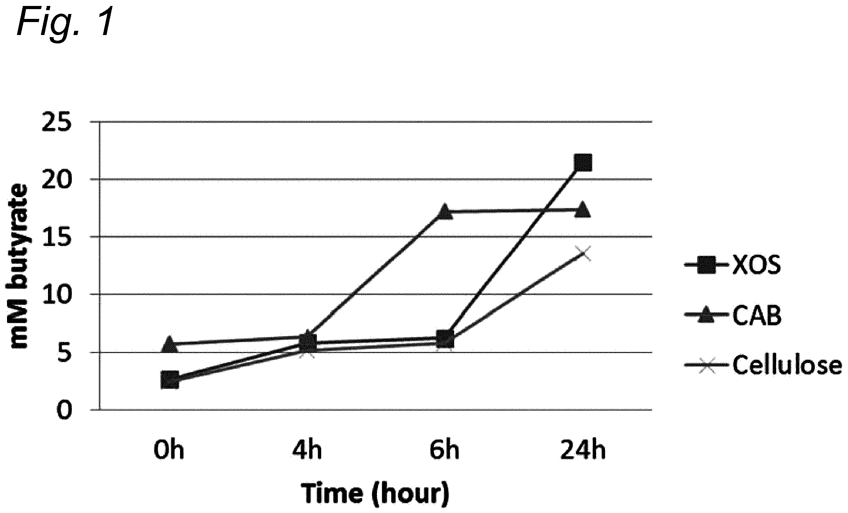

Figure 1 shows the butyrate concentrations measured for the ileum inoculum.

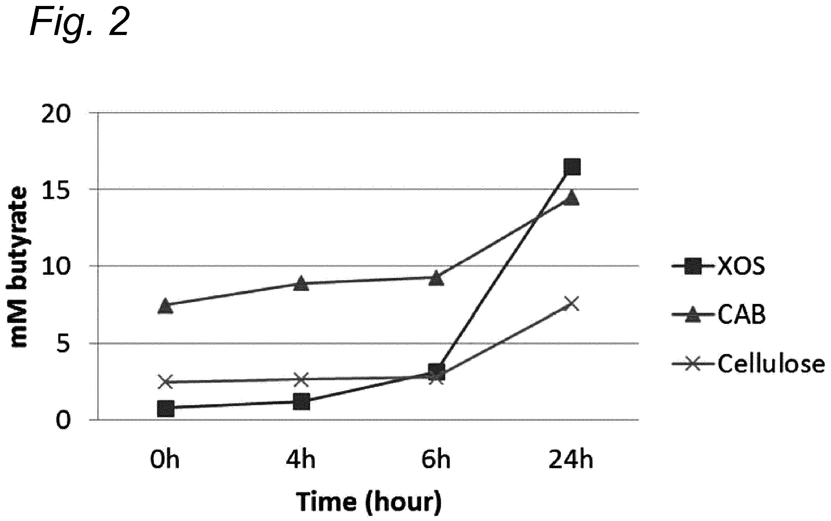

Figure 2 shows the butyrate concentrations measured for the colon inoculum.

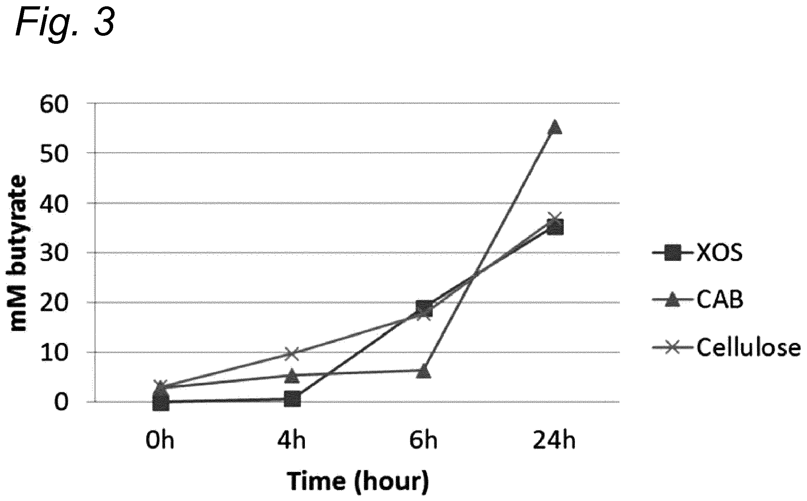

Figure 3 shows the butyrate concentrations measured for the caecum inoculum.

The results clearly show that CAB is able to increase the butyrate concentration significantly above the butyrate concentration measured for the negative control.

### Example 2: In vivo determination of butyrate concentration in poultry colon

In order to assess the capacity of the polysaccharide butyryl esters of the present invention to increase the butyrate concentration in the lower gastro-intestinal tract (GIT) of animals, an *in vivo* experiment was performed. A product in accordance with the invention was added to poultry feed, the butyrate concentration in the colon determined and compared to results obtained with other delayed release formulations or products.

### Animals

Ross 308 broiler chickens were used in this feed trial. 1-day old chicks were obtained from a commercial hatchery and were kept in isolation. All treatment groups were housed in the same room in separate cages, with litter on the floor. 60 chickens were divided into eight groups of 7 or 8 chickens each (including a blanco group).

### Test feed compositions

Seven different test feed compositions were prepared by mixing the test products shown in the below table with commercial mash broiler feed (Versele-Laga, Belgium) at a concentration of 3g sodium butyrate per kg feed. The test feed was subsequently pelletized (without steam) to avoid selective feed intake. The pelletizing technique, pellet size and commercial broiler feed was the same for all test products.

| **Test** | **Product** | **Supplier** | **Specifications** |
|---|---|---|---|
| 1 | Control - no additive | NA | NA |
| 2 | Adimix 30 Coated^{®} | Nutri-Ad^{®} | 30% sodium butyrate coated with palm fat |
| 3 | Polysaccharide butyryl ester: Cellulose acetate butyrate (CAB) | Acros Organics^{®} | 50-54% butyryl content |
| | | | <4% acetyl content |
| 4 | Polyhydroxybutyrate (*Ralstonia*) | Metabolix^{®} | 100% hydroxybutyrate |
| 5 | Wax matrix with 10% starch | Self-prepared | 30% sodium butyrate in Lunacera M^{®} crystalline matrix and starch. |
| 6 | Tributyrin | Self-prepared | 60% tributyrin (Proviron) on silica (Caldic, Belgium) |
| 7 | Unprotected butyrate (Sodium butyrate) | Nutri-Ad^{®} | 100% sodium butyrate |

### Experimental set-up

During the 19th, 20th and 21th days of age, average daily feed intake was measured by pen. Male and female chickens of 22 d of age were randomly assigned to one of the above described test feed compositions and received a restrictive feeding diet (95% of the average intake previously measured) during the 22-27 d period.

At 28 days of age, the birds were sacrificed and the colon collected.

### Measurement of butyrate concentrations

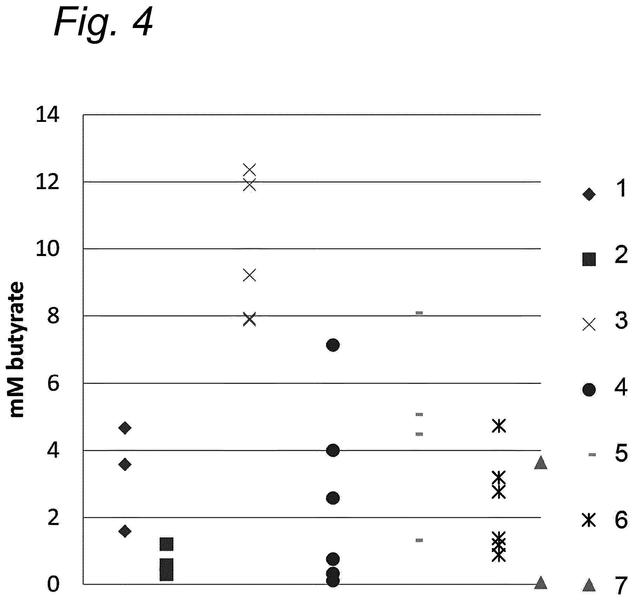

One gram of intestinal content of the colon of each chicken was weighed and diluted with 1mL distilled water. After dilution the samples were homogenized and centrifuged at 13.000 rpm for 20 minutes and stored at -20°C until extraction for HPLC-UV analysis. Extraction protocol and equipment was used as described by De Baere et al. (Journal of Pharmaceutical and Biomedical Analysis 80 (2013) 107- 115). For test 4, polyhydroxybutyrate, the hydroxybutyrate concentration was measured and displayed in Figure 4.

### Results

The results are shown in Figure 4 as a concentration of butyrate in millimolar (mM) / gram intestinal content plotted per treatment group. If there was not enough intestinal content or the extraction failed, no data was included for that measurement.

The results clearly show that CAB is able to increase the butyrate concentration in the colon significantly above the butyrate concentration measured for the control or other delayed release formulations or products.

### Example 3: In vivo determination of post-infection Salmonella presence in caeca

In order to assess the capacity of the polysaccharide butyryl esters of the present invention to decrease *Salmonella* colonization of the gut of animals infected by *Salmonella* Enteritidis, an *in vivo* experiment was performed. A product in accordance with the invention was added to poultry feed, the presence of *Salmonella* Enteritidis in the caecum determined and compared to results obtained with other delayed release butyrate formulations or products.

### Animals

Ross 308 broiler chickens were used in this trial. 1-day old chicks were obtained from a commercial hatchery. All treatment groups were housed under the same conditions in separate cages, with litter on the floor. 160 chickens were divided into eight groups of 20 chickens each (including the control group). The four test feed compositions were tested *in duplo.* All chicks had ad libitum access to water and feed.

### Test feed compositions

Four different test feed compositions were prepared by mixing the test products shown in the below table with commercial mash broiler feed (Versele-Laga, Belgium) at a concentration of 3g sodium butyrate per kg feed. The test feed was subsequently pelletized (without steam) to avoid selective feed intake. The pelletizing technique, pellet size and commercial broiler feed was the same for all test products.

| **Test** | **Product** | **Supplier** | **Specifications** |
|---|---|---|---|
| 1 | Control - no additive | NA | NA |
| 2 | Adimix 30 Coated^{®} | Nutri-Ad^{®} | 30% sodium butyrate coated with palm fat |
| 3 | Wax matrix with 10% starch | Self-prepared | 30% sodium butyrate in Lunacera M^{®} crystalline matrix and starch. |
| 4 | Poylsaccharide butyryl ester: Cellulose acetate butyrate (CAB) | Acros Organics^{®} | 50-54% butyryl content |
| | | | <4% acetyl content |

### Salmonella Strain

*Salmonella* Enteritidis strain SE147, a well-characterized streptomycin-resistant strain isolated from a poultry farm (Methner et al., 1995), was used in this experiment. The strain was grown for 6 h in Luria-Bertoni medium (LB, Sigma, St. Louis, MO), after which the number of cfu per milligram was determined by plating 10-fold dilutions of the bacterial suspension on xylose lysine deoxycholate agar (XLD, Oxoid, Basingstoke, UK) containing 100 µg/mL of streptomycin (Sigma, St. Louis, MO). The bacterial suspension was stored at 4°C during plate counting and was diluted in PBS to obtain the desired infection dose.

### Experimental set-up

At 17 days post-hatch all chicks were orally inoculated with 10^5 cfu of *Salmonella* Enteritidis per bird. At day 4 post infection all birds were euthanized and samples of caecum were taken for bacteriological analysis. The experiment was approved by the ethical committee of the Faculty of Veterinary Medicine, Ghent University.

### Bacteriological Analysis

Samples of caeca were homogenized in vol of buffered peptone water (BPW, Oxoid, Basingstoke, UK), and 10-fold dilutions were made in PBS. For each dilution, 6 × 20 µL was inoculated on XLD plates containing 100 µg/mL streptomycin. After an overnight incubation at 37°C, the number of cfu per gram of tissue was determined. Samples that were negative after direct plating were preenriched overnight at 37°C in buffered peptone water (BPW, Oxoid, Basingstoke, UK), after which all samples were enriched by addition of 1 mL of this suspension to 9 mL of brilliant green tetrathionate broth (Oxoid, Basingstoke, UK). Samples that were Salmonella negative after titration but positive after enrichment were assumed to contain 1 × 10^1 cfu/g of tissue. Samples that were still negative after enrichment were assumed to have 0 cfu/g of tissue.

### Results

As shown in the table below, administration of feed comprising a polysaccharide butyryl ester such as CAB provides significantly improved results with regard to *Salmonella* colonization of the caeca compared to other delayed release formulations or products.

| ***Salmonella* presence** | **Control** | **2** | **3** | **4** |
|---|---|---|---|---|
| Negative | 0 | 0 | 0 | 0 |
| Positive after enrichment | 6 | 14 | 13 | 27 |
| 10² < x < 10³ cfu/g | 12 | 8 | 7 | 4 |
| 10³ < x < 10⁴ cfu/g | 6 | 14 | 5 | 8 |
| > 10⁴ cfu/g | 15 | 4 | 15 | 1 |

### Example 4: In vivo determination of post-infection Salmonella presence in cloacal swabs

In order to assess the capacity of the polysaccharide butyryl esters of the present invention to decrease *Salmonella* fecal shedding of animals infected by *Salmonella* Enteritidis, an *in vivo* experiment was performed. A product as described above was added to poultry feed, the presence of *Salmonella* Enteritidis in cloacal swabs determined and compared to results obtained with other delayed release butyrate formulations or products.

### Animals

Ross 308 broiler chickens were used in this trial. 1-day old chicks were obtained from a commercial hatchery. All treatment groups were housed under the same conditions in separate cages, with litter on the floor. 80 chickens were divided into four groups of 20 chickens each (including the control group). The four test feed compositions listed below were tested. All chicks had ad libitum access to water and feed.

### Test feed compositions

Four different test feed compositions were prepared by mixing the test products shown in the below table with commercial mash broiler feed (Versele-Laga, Belgium) at a concentration of 3g sodium butyrate per kg feed. The test feed was subsequently pelletized (without steam) to avoid selective feed intake. The pelletizing technique, pellet size and commercial broiler feed was the same for all test products.

| **Test** | **Product** | **Supplier** | **Specifications** |
|---|---|---|---|
| 1 | Control - no additive | NA | NA |
| 2 | Adimix 30 Coated^{®} | Nutri-Ad^{®} | 30% sodium butyrate coated with palm fat |
| 3 | Wax matrix with 10% starch | Self-prepared | 30% sodium butyrate in Lunacera M^{®} crystalline matrix and starch. |
| 4 | Polsaccharide butyryl ester: Cellulose acetate butyrate (CAB) | Acros Organics^{®} | 50-54% butyryl content |
| | | | <4% acetyl content |

### Salmonella Strain

*Salmonella Enteritidis* strain SE147, a well-characterized streptomycin-resistant strain isolated from a poultry farm (Methner et al., 1995), was used in this experiment. The strain was grown for 6 h in Luria-Bertoni medium (LB, Sigma, St. Louis, MO), after which the number of cfu per milligram was determined by plating 10-fold dilutions of the bacterial suspension on xylose lysine deoxycholate agar (XLD, Oxoid, Basingstoke, UK) containing 100 µg/mL of streptomycin (Sigma, St. Louis, MO). The bacterial suspension was stored at 4°C during plate counting and was diluted in PBS to obtain the desired infection dose.

### Experimental set-up

At 17 days post-hatch all chicks were orally inoculated with 10^5 cfu of *Salmonella* Enteritidis per bird. At day -1, 1 and 3 post infection, cloacal swabs were taken for bacteriological analysis. The experiment was approved by the ethical committee of the Faculty of Veterinary Medicine, Ghent University.

### Bacteriological Analysis

Cloacal swabs were plated on XLD plates containing 100 µg/mL of streptomycin and incubated overnight at 37°C. Samples that were negative after direct plating were preenriched overnight at 37°C in buffered peptone water (BPW, Oxoid, Basingstoke, UK), after which the samples were enriched by addition of 1 mL of this suspension to 9 mL of brilliant green tetrathionate broth (Oxoid, Basingstoke, UK). This suspension was again incubated overnight at 37°C, and afterwards plated on XLD containing 100 µg/mL of streptomycin. After an overnight incubation at 37°C, the number of cfu per cloacal swab was determined. Samples that were *Salmonella* negative after titration but positive after enrichment were marked as positive for *Salmonella* presence. Samples that were still negative after enrichment were marked as negative for *Salmonella* presence.

### Results

As shown in the table below, administration of feed comprising a polysaccharide butyryl ester such as CAB provides significantly improved results with regard to fecal shedding of *Salmonella* compared to other delayed release formulations or products.

| **Days past infection** | **Salmonella presence** | **Control** | **2** | **3** | **4** |
|---|---|---|---|---|---|
| -1 | Negative | 20 | 20 | 20 | 20 |
| | Positive | 0 | 0 | 0 | 0 |
| 1 | Negative | 5 | 14 | 17 | 16 |
| | Positive | 15 | 6 | 3 | 4 |
| 3 | Negative | 5 | 14 | 13 | 17 |
| | Positive | 15 | 6 | 7 | 3 |

### Example 5: In vivo determination of growth performance

In order to assess the capacity of the polysaccharide butyryl esters of the present invention to influence the growth performance under suboptimal farming practices, an *in vivo* experiment was performed. A product as described above was added to a challenging rapeseed meal (RSM) diet, the effects on growth performance determined and compared to results obtained with other delayed release butyrate formulations or products.

### Experimental design

The experiment was designed as a complete randomized block design. Treatment groups were arranged as a 3 x 2 factorial plus control with 3 delayed release butyrate formulations or products and two supplementation levels (0.25 or 1 g butyrate/kg feed as fed basis). Each treatment group was replicated six times, resulting in a total of six blocks. Diets were based on a RSM-Corn-Wheat starter, grower and finisher diets detailed in the below tables. Experimental starter and grower diets were derived from the basal recipe by adding the feed additives at the expense of soybean oil.

| | **Product** | **Supplier** | **Specifications** |
|---|---|---|---|
| 1 | Control - no additive | NA | NA |
| 2 | Adimix 30 Coated^{®} | Nutri-Ad^{®} | 30% sodium butyrate coated with palm fat |
| 3 | Wax matrix | Self-prepared | 30% sodium butyrate in Lunacera M^{®} crystalline matrix. |
| 4 | Polsaccharide butyryl ester: Cellulose acetate butyrate (CAB) | Acros Organics^{®} | 50-54% butyryl content |
| | | | <4% acetyl content |

### Starter composition

| Item | | 1-CTR | 2-H | 2-L | 3-H | 3-L | 4-H | 4-L |
|---|---|---|---|---|---|---|---|---|
| Ingredient | | | | | | | | |
| | Corn | 295.96 | 295.96 | 295.96 | 295.96 | 295.96 | 295.96 | 295.96 |
| | Wheat | 200 | 200 | 200 | 200 | 200 | 200 | 200 |
| | Rapeseedmeal | 350 | 350 | 350 | 350 | 350 | 350 | 350 |
| | Fishmeal | 75 | 75 | 75 | 75 | 75 | 75 | 75 |
| | Soybean oil | 55.29 | 51.96 | 54.46 | 51.96 | 54.46 | 53.29 | 54.79 |
| | Premix | 5 | 5 | 5 | 5 | 5 | 5 | 5 |
| | Lime fine | 9 | 9 | 9 | 9 | 9 | 9 | 9 |
| | Monocalcium phosphate | 5.5 | 5.5 | 5.5 | 5.5 | 5.5 | 5.5 | 5.5 |
| | Salt | 0.9 | 0.9 | 0.9 | 0.9 | 0.9 | 0.9 | 0.9 |
| | NaHCO3 | 2.8 | 2.8 | 2.8 | 2.8 | 2.8 | 2.8 | 2.8 |
| | L-Lysine HCl | 0.5 | 0.5 | 0.5 | 0.5 | 0.5 | 0.5 | 0.5 |
| | Natuphos 1000G | 0.05 | 0.05 | 0.05 | 0.05 | 0.05 | 0.05 | 0.05 |
| | Adimix 30 Coated^{®} | 0 | 3.33 | 0.83 | 0 | 0 | 0 | 0 |
| | Wax matrix | 0 | 0 | 0 | 3.33 | 0.83 | 0 | 0 |
| | CAB | 0 | 0 | 0 | 0 | 0 | 2 | 0.5 |
| | Total | 1000 | 1000 | 1000 | 1000 | 1000 | 1000 | 1000 |

### Grower composition

| Item | | 1-CTR | 2-H | 2-L | 3-H | 3-L | 4-H | 4-L |
|---|---|---|---|---|---|---|---|---|
| Ingredient | | | | | | | | |
| | Corn | 215.35 | 215.35 | 215.35 | 215.35 | 215.35 | 215.35 | 215.35 |
| | Wheat | 300 | 300 | 300 | 300 | 300 | 300 | 300 |
| | Rapeseedmeal | 350 | 350 | 350 | 350 | 350 | 350 | 350 |
| | Fishmeal | 55 | 55 | 55 | 55 | 55 | 55 | 55 |
| | Soybean oil | 63.00 | 59.67 | 62.17 | 59.67 | 62.17 | 61.00 | 62.50 |
| | Premix | 5 | 5 | 5 | 5 | 5 | 5 | 5 |
| | Lime fine | 8 | 8 | 8 | 8 | 8 | 8 | 8 |
| | Salt | 1.2 | 1.2 | 1.2 | 1.2 | 1.2 | 1.2 | 1.2 |
| | NaHCO3 | 1.4 | 1.4 | 1.4 | 1.4 | 1.4 | 1.4 | 1.4 |
| | L-Lysine HCl | 1 | 1 | 1 | 1 | 1 | 1 | 1 |
| | Natuphos 1000G | 0.05 | 0.05 | 0.05 | 0.05 | 0.05 | 0.05 | 0.05 |
| | Adimix 30 Coated^{®} | 0 | 3.33 | 0.83 | 0 | 0 | 0 | 0 |
| | Wax matrix | 0 | 0 | 0 | 3.33 | 0.83 | 0 | 0 |
| | CAB | 0 | 0 | 0 | 0 | 0 | 2 | 0.5 |
| | Total | 1000 | 1000 | 1000 | 1000 | 1000 | 1000 | 1000 |

### Finisher composition

| Item | | 1-CTR |
|---|---|---|
| Ingredient | | |
| | Corn | 229.35 |
| | Wheat | 300 |
| | Rapeseedmeal | 350 |
| | Fishmeal | 40 |
| | Soybean oil | 64.00 |
| | Premix | 5 |
| | Lime fine | 7.5 |
| | Salt | 1.2 |
| | NaHCO3 | 1.4 |
| | L-Lysine HCl | 1.5 |
| | Natuphos 1000G | 0.05 |
| | Fat-coated butyrate | 0 |
| | Wax-coated butyrate | 0 |
| | CAB | 0 |
| | Total | 1000 |

The premix provided per kg of diet: vitamin A, 12.000 IU; vitamin D3, 2.500 IU; vitamin E, 50 mg; vitamin B2, 7.5 mg; vitamin B6, 3.5 mg; vitamin B1, 2.0 mg; vitamin K3, 1.5 mg; vitamin B12, 20 µg; choline chloride, 460 mg; antioxidant (oxytrap PXN),125 mg; niacin, 35 mg; pantothenic acid, 12 mg; biotin, 0.2 mg; folic acid, 1 mg; Mn, 85 mg; Fe, 80 mg; Zn, 60 mg; Cu, 12 mg; I, 0.8 mg; Se, 0.15 mg.

Starter diets were fed from d 0 till d 21, grower diets from d 22 till d 35 and finisher diet was fed from d 36 till slaughter at d 42. Diets were formulated to meet or exceed the requirements of broiler chickens and were produced by Research Diet Services (Wijk bij Duurstede, The Netherlands).

### Birds and experimental procedures

The experiment was conducted at the research farm Carus of Wageningen University. A total of 357 male one-day-old broilers (initial BW 47 g; Ross 308, Aviagen Group, Newbridge, United Kingdom) were obtained from a commercial hatchery (Morren Breeders B.V., Lunteren, the Netherlands). Upon arrival, birds were individually weighed and assigned to a weighed category (Small < µ - 0.68 x σ; µ - 0.68 x σ < Medium < µ + 0.68 x σ; Large > µ + 0.68 x σ). Birds of each category were randomly assigned to one of the 42 floor pen of two climate-controlled rooms. Each pen housed 8 to 9 birds, had a dimension of 1.85 x 1 m (L x W) and was enriched with a perch. Wood shavings were used as a bedding material. Ambient temperature was maintained at 32°C until d 3 and thereafter gradually reduced to 22°C at d 23. A 23L:1D photoperiod was applied until d 3 and was changed thereafter to 16L:8D. Birds were allowed *ad libitum* access to feed and water. Pen bodyweights as well as pen feed intake were recorded at d 21, 35 and 42. Mortality was monitored on a daily basis and body weight of dead birds was recorded.

### Results

The tables below show the feed conversion ratio (FCR), average bodyweight gain (ADG) and average daily feed intake (ADFI) and mortality rate. FCR was calculated at pen level using ADG and ADFI. Results were also recalculated as percentage of change relative to their respective control.

The tables below show the mortality, average daily weight gain (ADG), average daily food intake (ADFI) and feed conversion ratio (FCR) results obtained in this experiment. The data shows that feed supplementation with a polysaccharide butyryl ester such as CAB provides superior growth performance results compared to other delayed release butyrate formulations or products. The polysaccharide butyryl ester in accordance with the present invention provides the highly desirable combination of improved ADG and ADFI combined with decreased FCR and mortality during the supplementation period without showing adverse effects during the finisher period.

The results also show that in the starter and grower phase, increasing inclusion of the polysaccharide butyryl ester (4-L vs. 4-H) leads to increased performance (in contrast to the effect observed for the wax compositions 3-L vs. 3-H).

Effect of dietary treatments on performance parameters during the starter, grower and finisher periods.

| | Dietary treatment | | | | | | |
|---|---|---|---|---|---|---|---|
| | 1 | 2 | | 3 | | 4 | |
| Item | CTR | L | H | L | H | L | H |
| **0-21 d** | | | | | | | |
| Mortality, % | 5.9 | 2.0 | 0.0 | 0.0 | 0.0 | 0.0 | 0.0 |
| ADG, g/bird | 39.9 | 39.7 | 42.0 | 41.8 | 40.4 | 40.6 | 41.4 |
| ADFI, g/bird | 54.5 | 53.8 | 56.7 | 56.6 | 54.2 | 55.3 | 56.9 |
| FCR, g:g | 1.37 | 1.36 | 1.35 | 1.35 | 1.34 | 1.36 | 1.38 |

| **22-35 d** | | | | | | | |
|---|---|---|---|---|---|---|---|
| Mortality, % | 0.0 | 0.0 | 3.9 | 3.9 | 2.0 | 0.0 | 0.0 |
| ADG, g/bird | 89.8 | 90.8 | 90.5 | 89.4 | 87.6 | 93.8 | 96.4 |
| ADFI, g/bird | 140.3 | 139.9 | 154.0 | 147.0 | 139.0 | 141.0 | 146.3 |
| FCR, g:g | 1.58 | 1.55 | 1.73 | 1.65 | 1.59 | 1.51 | 1.52 |

| **35-42 d** | | | | | | | |
|---|---|---|---|---|---|---|---|
| Mortality, % | 4.2 | 0.0 | 0.0 | 2.0 | 4.0 | 0.0 | 2.0 |
| ADG, g/bird | 131.9 | 107.3 | 125.0 | 120.3 | 116.4 | 115.7 | 116.9 |
| ADFI, g/bird | 224.1 | 197.6 | 211.5 | 198.6 | 214.7 | 213.0 | 211.2 |
| FCR, g:g | 1.71 | 1.85 | 1.71 | 1.66 | 1.84 | 1.85 | 1.81 |

Effect of dietary treatments on performance parameters during the supplementation and total experimental periods.

| | Dietary treatment | | | | | | |
|---|---|---|---|---|---|---|---|
| | 1 | 2 | | 3 | | 4 | |
| Item | CTR | L | H | L | H | L | H |
| Supplementation period (0-35 d) | | | | | | | |
| Mortality, % | 7.8 | 2.0 | 3.9 | 5.9 | 3.9 | 0.0 | 0.0 |
| ADG, g/bird | 59.51 | 60 | 61.3 | 60.8 | 59.1 | 61.9 | 63.4 |
| ADFI, g/bird | 88.26 | 88 | 95.5 | 92.5 | 87.8 | 89.6 | 92.7 |
| FCR, g:g | 1.49 | 1.47 | 1.56 | 1.53 | 1.49 | 1.45 | 1.46 |

| Total experiment (0-42 d) | | | | | | | |
|---|---|---|---|---|---|---|---|
| Mortality, % | 9.8 | 2.0 | 3.9 | 5.9 | 5.9 | 0.0 | 2.0 |
| ADG, g/bird | 70.42 | 67.9 | 71.6 | 70.3 | 68.4 | 70.9 | 72.3 |
| ADFI, g/bird | 108.9 | 106 | 114 | 110 | 108 | 110 | 112 |
| FCR, g:g | 1.55 | 1.57 | 1.60 | 1.56 | 1.58 | 1.56 | 1.56 |

### Effect of dietary treatments on the performance parameters recalculated as percentage of change relative to their respective control during the supplementation period (0-35 d)

| | 2 | | 3 | | 4 | |
|---|---|---|---|---|---|---|
| | L | H | L | H | L | H |
| ADG, % change | 0.9 | 3.0 | 2.0 | -0.7 | 3.8 | 6.1 |
| ADFI, % change | -0.3 | 7.6 | 4.6 | -0.6 | 1.5 | 4.7 |
| FCR, % change | -1.4 | 4.8 | 2.3 | -0.2 | -2.7 | -1.8 |

### Example 6: In vivo determination of feed conversion rate

In order to assess the capacity of the polysaccharide butyryl esters of the present invention to influence the feed conversion rate under suboptimal farming practices, an *in vivo* experiment was performed. A product as described above was added to a challenging rapeseed meal (RSM) diet, the effects on feed conversion rate determined, statistically analyzed and compared to results obtained with other delayed release (hydroxy)butyrate formulations or products.

### Experimental design

The experiment was designed as a complete randomized block design. Treatment groups were arranged as 10 birds/pen x 8 replicates x 4 treatments: a control and 3 delayed release butyrate formulations or products (at 1 g (hydroxy)butyrate/kg feed). Diets were based on a RSM-Corn-Wheat starter and grower diets detailed in the below tables. Experimental starter and grower diets were derived from the basal recipe by adding the feed additives at the expense of soybean oil.

| | **Product** | **Supplier** | **Specifications** |
|---|---|---|---|
| 1 | Control - no additive | NA | NA |
| 2 | Adimix 30 Coated^{®} | Nutri-Ad^{®} | 30% sodium butyrate coated with palm fat |
| 3 | Polyhydroxybutyrate (PHB) | Nutri-Ad^{®} | 0.10% butyrate biomass |
| 4 | Polysaccharide butyryl ester: Cellulose acetate butyrate (CAB) | Fisher-Scientific, Waltham, USA | 50-54% butyryl content <4% acetyl content |

### Starter composition

| Item | | 1-CTR | 2 | 3 | 4 |
|---|---|---|---|---|---|
| Ingredient | | | | | |
| | Corn | 296 | 296 | 296 | 296 |
| | Wheat | 200 | 200 | 200 | 200 |
| | Rapeseedmeal | 350 | 350 | 350 | 350 |
| | Fishmeal | 75 | 75 | 75 | 75 |
| | Soybean oil | 55.3 | 52.0 | 54.3 | 53.5 |
| | Premix | 5 | 5 | 5 | 5 |
| | Lime fine | 9 | 9 | 9 | 9 |
| | Monocalcium phosphate | 5.5 | 5.5 | 5.5 | 5.5 |
| | Salt | 0.9 | 0.9 | 0.9 | 0.9 |
| | NaHCO3 | 2.8 | 2.8 | 2.8 | 2.8 |
| | L-Lysine HCl | 0.5 | 0.5 | 0.5 | 0.5 |
| | Natuphos 1000G | 0.05 | 0.05 | 0.05 | 0.05 |
| | Adimix 30 Coated^{®} | 0 | 3.33 | 0 | 0 |
| | PHB | 0 | 0 | 1 | 0 |
| | CAB | 0 | 0 | 0 | 1.8 |
| | Total | 1000 | 1000 | 1000 | 1000 |

### Grower composition

| Item | | 1-CTR | 2 | 3 | 4 |
|---|---|---|---|---|---|
| Ingredient | | | | | |
| | Corn | 215.4 | 215.4 | 215.4 | 215.4 |
| | Wheat | 300.0 | 300.0 | 300.0 | 300.0 |
| | Rapeseedmeal | 350.0 | 350.0 | 350.0 | 350.0 |
| | Fishmeal | 55.0 | 55.0 | 55.0 | 55.0 |
| | Soybean oil | 63.0 | 59.7 | 62.0 | 61.2 |
| | Premix | 5 | 5 | 5 | 5 |
| | Lime fine | 8 | 8 | 8 | 8 |
| | Monocalcium phosphate | - | - | - | - |
| | Salt | 1.2 | 1.2 | 1.2 | 1.2 |
| | NaHCO3 | 1.4 | 1.4 | 1.4 | 1.4 |
| | L-Lysine HCl | 1 | 1 | 1 | 1 |
| | Natuphos 1000G | 0.05 | 0.05 | 0.05 | 0.05 |
| | Adimix 30 Coated^{®} | 0 | 3.33 | 0.00 | 0 |
| | PHB | 0 | 0 | 1 | 0 |
| | CAB | 0 | 0 | 0 | 1.8 |
| | Total | 1000 | 1000 | 1000 | 1000 |

The premix provided per kg of diet: vitamin A, 12.000 IU; vitamin D3, 2.500 IU; vitamin E, 50 mg; vitamin B2, 7.5 mg; vitamin B6, 3.5 mg; vitamin B1, 2.0 mg; vitamin K3, 1.5 mg; vitamin B12, 20 µg; choline chloride, 460 mg; antioxidant (oxytrap PXN),125 mg; niacin, 35 mg; pantothenic acid, 12 mg; biotin, 0.2 mg; folic acid, 1 mg; Mn, 85 mg; Fe, 80 mg; Zn, 60 mg; Cu, 12 mg; I, 0.8 mg; Se, 0.15 mg.

Starter diets were fed from d 0 till d 21 and grower diets from d 22 till d 35. Diets were formulated to meet or exceed the requirements of broiler chickens and were produced by Research Diet Services (Wijk bij Duurstede, The Netherlands).

### Birds and experimental procedures

The experiment was conducted at the experimental poultry house 12 of the ILVO-DIER, (Burg, Merelbeke, BE). A total of 320 male one-day-old broilers (Ross 308, Aviagen Group, Newbridge, United Kingdom) were obtained from a commercial hatchery (Belgabroed NV, Merksplas, BE). Upon arrival, birds were randomly assigned to one of the 32 floor pens of a single climate-controlled room. Each pen housed 10 birds and had a dimension of 2.1 x 1 m. Ambient temperature was maintained at 29-30 °C until d 7 and thereafter reduced by 2 °C per week. A 23L:1D photoperiod was applied until d 7 and was changed thereafter to 16L:8D. Birds were allowed ad libitum access to feed and water. Individual and pen body weights, as well as pen feed intake, were recorded every week.

### Statistical analysis

Performance parameters were analysed using the PROC GLM of SAS (version 9.3, SAS Institute Inc., Cary, NC) using the following model ${Y}_{ijk} = μ + {D}_{i} + {W}_{j} + {B}_{k} + {AW}_{ij} + {ε}_{ijk}$ where Y*ᵢⱼₖ* is the observed response of the *k*^{th} replicate (*k* = 1 to 8) of the fed the *i*^{th} diet (*i* = CTR, FCB, PHB, CAB) during the *j*^{th} week (*j* = 1 to 5), D*ᵢ* is the *i*^{th} fixed diet effect, B*ₖ* is the *k*^{th} fixed block effect, W*ⱼ* is the *j*^{th} random effect of measurement period, and AD*ᵢⱼ* the interaction effect between the diet and measurement period, and *εᵢⱼₖ* is the residual error term of the *k*^{th} replicate fed the *i*^{th} diet at the *j*^{th} measurement period. When a significant diet effect was detected, means were separated using Tukey post-hoc test. When a significant interaction between diet and measurement period was detected, means were separated using Tukey post-hoc test and the interaction term was partitioned per week by a simple effect test.

### Results

Effects of dietary butyrate supplementation on the feed conversion ratio (FCR) over the 0-35 d supplementation period are shown in the table below.

The data shows that feed supplementation with a polysaccharide butyryl ester such as CAB provides superior feed conversion ratio results compared to other delayed release butyrate formulations or products. The feed conversion ratio improvement for CAB was statistically significant.

### Effect of dietary butyrate supplementation on the growth performance during the entire 0-35 d period

| Item | Treatment group | | | | Pooled SEM | p-value | | | |
|---|---|---|---|---|---|---|---|---|---|
| | 1-CTR | 2 | 3 | 4 | | Diet | Week | Block | Diet*Week |
| BWG | 48.41 | 48.26 | 48.36 | 48.56 | 1.62 | 0.9981 | <.0001 | 0.2511 | **0.0032** |
| VFI | 79.15 | 76.52 | 77.42 | 76.08 | 1.93 | 0.6898 | <.0001 | 0.5882 | 0.5192 |
| FCR | 1.64 | 1.59 | 1.60 | 1.57 | 0.02 | **0.0164** | <.0001 | 0.7997 | **0.0194** |

## Claims

1. A cellulose butyryl ester which further comprises acetyl ester groups wherein the cellulose butyryl ester is **characterized by** a number average molar mass of 2000-1000000 g/mol, preferably 5000-500000 g/mol, preferably 7000-250000 g/mol, preferably 10000-100000 g/mol, preferably 12000-50000 g/mol, preferably 13000-25000 g/mol, most preferably 15000-17000 g/mol, for use in a prophylactic or curative method of treatment.

2. The cellulose butyryl ester for use according to claim 1 wherein the average number of butyryl groups per monosaccharide unit is within the range of 0.1-4, preferably 1-3.5, preferably 1.75-3.25, preferably 2.25-3, preferably 2.5-2.95.

3. The cellulose butyryl ester for use according to claim 1 or 2, wherein the cellulose butyryl ester is **characterized by** an acetyl content of at least 1 wt.% by total weight of the cellulose butyryl ester, preferably at least 2 wt.%, preferably at least 3 wt.%, preferably at least 4 wt.%.

4. The cellulose butyryl ester for use according to any one of claims 1 to 3 in the prophylaxis or treatment of pathogen infection, preferably by one or more of bacteria, eimeria, viruses and fungi, preferably bacteria, preferably one or more of *Clostridium acetobutylicum, Escherichia coli, Streptococcus cremoris, Lactococcus lactis, Lactococcus cremoris, Clostridium perfringens, Campylobacter jejuni, Campylobacter coli, Lawsonia intracellullaris, Brachyspira hyodysenteriae, Enterococcus caecorum, Streptococcus suis* and *Salmonella enteritidis.*

5. The cellulose butyryl ester for use according to any one of claims 1 to 4, for improving the intestinal microflora.

6. The cellulose butyryl ester for use according to any one of claims 1 to 5, in a treatment of livestock animals such as poultry species.

## Patentansprüche

1. Cellulose-Butyrylester, der ferner Acetylestergruppen umfasst, wobei der Cellulose-Butyrylester durch eine zahlenmittlere Molmasse von 2000-1000000 g/mol, vorzugsweise 5000-500000 g/mol, vorzugsweise 7000-250000 g/mol, vorzugsweise 10000-100000 g/mol, vorzugsweise 12000-50000 g/mol, vorzugsweise 13000-25000 g/mol, vorzugsweise 15000-17000 g/mol, gekennzeichnet ist, zur Verwendung in einem prophylaktischen oder kurativen Behandlungsverfahren.

2. Cellulose-Butyrylester zur Verwendung nach Anspruch 1, wobei die durchschnittliche Anzahl der Butyrylgruppen pro Monosaccharideinheit im Bereich von 0,1-4, vorzugsweise 1-3,5, vorzugsweise 1,75-3,25, vorzugsweise 2,25-3, vorzugsweise 2,5-2,95, liegt.

3. Cellulose-Butyrylester zur Verwendung nach Anspruch 1 oder 2, wobei der Cellulose-Butyrylester durch einen Acetylgehalt von mindestens 1 Gew.-% des Gesamtgewichts des Cellulose-Butyrylesters, vorzugsweise mindestens 2 Gew.-%, vorzugsweise mindestens 3 Gew.-%, vorzugsweise mindestens 4 Gew.-%, gekennzeichnet ist.

4. Cellulose-Butyrylester zur Verwendung nach einem der Ansprüche 1 bis 3 zur Prophylaxe oder Behandlung einer pathogenen Infektion, vorzugsweise durch eines oder mehrere von Bakterien, Eimeria, Viren und Pilzen, vorzugsweise Bakterien, vorzugsweise eines oder mehrere von *Clostridium acetobutylicum, Escherichia coli, Streptococcus cremoris, Lactococcus lactis, Lactococcus cremoris, Clostridium perfringens, Campylobacter jejuni, Campylobacter coli, Lawsonia intracellullaris, Brachyspira hyodysenteriae, Enterococcus caecorum, Streptococcus suis* un*d Salmonella enteritidis.*

5. Cellulosebutyrylester zur Verwendung nach einem der Ansprüche 1 bis 4 zur Verbesserung der Darmmikroflora.

6. Cellulosebutyrylester zur Verwendung nach einem der Ansprüche 1 bis 5 bei der Behandlung von Nutztieren wie Geflügelarten.

## Revendications

1. Ester butyrique de cellulose qui comprend en outre des groupes ester d'acétyle où l'ester butyrique de cellulose est **caractérisé par** une masse molaire moyenne en nombre allant de 2000 à 1000000 g/mol, de préférence de 5000 à 500000 g/mol, de préférence de 7000 à 250000 g/mol, de préférence de 10000 à 100000 g/mol, de préférence de 12000 à 50000 g/mol, de préférence de 13000 à 25000 g/mol, plus préférentiellement de 15000 à 17000 g/mol, pour une utilisation dans un procédé de traitement prophylactique ou curatif.

2. Ester butyrique de cellulose pour une utilisation selon la revendication 1, dans lequel le nombre moyen de groupes butyryle par unité monosaccharide se trouve dans la plage allant de 0,1 à 4, de préférence de 1 à 3,5, de préférence de 1,75 à 3,25, de préférence de 2,25 à 3, de préférence de 2,5 à 2,95.

3. Ester butyrique de cellulose pour une utilisation selon la revendication 1 ou 2, dans lequel l'ester butyrique de cellulose est **caractérisé par** une teneur en acétyle d'au moins 1% en poids par rapport au poids total de l'ester butyrique de cellulose, de préférence d'au moins 2% en poids, de préférence d'au moins 3% en poids, de préférence d'au moins 4% en poids.

4. Ester butyrique de cellulose pour une utilisation selon l'une quelconque des revendications 1 à 3 dans la prophylaxie ou le traitement d'une infection par un agent pathogène, de préférence par un ou plusieurs parmi les bactéries, les eimeria, les virus et les champignons, de préférence les bactéries, de préférence un ou plusieurs parmi *Clostridium acetobutylicum*, *Escherichia coli*, *Streptococcus cremoris*, *Lactococcus lactis*, *Lactococcus cremori*s, *Clostridium perfringens*, *Campylobacter jejuni*, *Campylobacter coli*, *Lawsonia intracellullaris*, *Brachyspira hyodysenteriae*, *Enterococcus caecorum*, *Streptococcus suis* et *Salmonella enteritidis*.

5. Ester butyrique de cellulose pour une utilisation selon l'une quelconque des revendications 1 à 4, pour améliorer la microflore intestinale.

6. Ester butyrique de cellulose pour une utilisation selon l'une quelconque des revendications 1 à 5, dans un traitement d'animaux d'élevage tels que des espèces de volailles.
